# EUROPEAN PATENT SPECIFICATION

(11) **EP 2 002 946 B1**
(45) Date of publication and mention of the grant of the patent: **30.12.2009**
(21) Application number: 07425368.3
(22) Date of filing: 12.06.2007
(51) Int. Cl.: B25J 13/08

(54) **Method for the generation of intrinsically pulse-coded sensorial information and biomimetic sensor for robotics and prosthetics operating according to this method**
Verfahren zur Erzeugung intrinsisch impulskodierter sensorischer Informationen und entsprechend diesem Verfahren arbeitender biomimetischer Sensor für Robotersysteme und Prothesen
Procédé pour la génération d'informations sensorielles intrinsèquement codées par impulsion et capteur biomimétique pour la robotique et prothèses fonctionnant selon ce procédé

(43) Date of publication of application: 17.12.2008
(73) Proprietor: Scuola Superiore di Studi Universitari e di Perfezionamento Sant'Anna, 56125 Pisa (IT)
(72) Inventor: Izzo, Ivano, 56121 Pisa (IT); Ascari, Luca, 56025 Pontedera (Pisa) (IT)
(74) Representative: Bardini, Marco Luigi

(56) References cited:
- JP-A- 2 049 167
- DARIO P ET AL: "Design, fabrication and applications of biomimetic sensors in biorobotics" INFORMATION ACQUISITION, 2005 IEEE INTERNATIONAL CONFERENCE ON HONG KONG AND MACAU, CHINA JUNE 27 - JULY 3, 2005, PISCATAWAY, NJ, USA,IEEE, 27 June 2005 (2005-06-27), pages 263-266, XP010918554 ISBN: 0-7803-9303-1
- MARTIN J. PEARSON, IAN GILHESPY, CHRIS MELHUISH, BEN MITCHINSON, MOKTHAR NIBOUCHE, ANTHONY G. PIPE, TONY J. PRESCOTT: "A Biomimetic Haptic Sensor" International Journal of Advanced Robotic Systems Vienna Univ. of Technol Austria, vol. 2, no. 4, December 2005 (2005-12), pages 335-343, XP002470615 ISSN: 1729-8806

## Description

### Field of the Invention

The present invention relates generally to the field of robotics and prosthetic applications and, more precisely, relates to a method for the generation of intrinsically pulse-coded sensorial information and to a biomimetic sensor for robotics and prosthetics, operating according to said method.

### State of the art

One of the greatest problems that lie at the basis of the failed widespread diffusion of robots in daily life is the inability to perceive the environment in which they move, made up of persons, objects and spaces, organically and without "grey areas". A robot, for example humanoid, which were to move among people without taking account of the possible impacts caused by its own or others' movements would pose a danger and could not be tolerated. The most obvious solutions consist of covering the entire external surface of a robot with a "sensitive skin", like human skin.

The consequent problems are mainly of two types: a) supplying electric power to each individual sensor and extracting the signal from each of them, with a consequent high number of electrical wires, and b) how to handle effectively large amount of data coming from the sensors.

As far as the second point is concerned, analysis of the strategies used in the nervous systems in the animal world, strategies of proven efficiency also with a very high number of signals, has provided the inspiration for a series of architectures of processing of biomorphic information (both hardware and software) in which this information is "pulse-coded" (spiking). The first spiking architectures to be introduced were the PCNN (pulse-coded neural networks), artificial neural software networks in which communication between the "neurons" takes place via "pulses" instead of via logical values. This led to investigation and publication of a series of learning algorithms on these architectures, algorithms in many cases inspired by the world of biology, in which spike-based communication is at the centre of nervous systems (in this respect see, for example: Belatreche, A. et al., Advances in design and application of spiking neural networks, Soft Computing, 11 (3): 239-248 Feb 2007; Amin, HH. et al., Learning algorithm for spiking neural networks, Advances in Natural Computation, PT 1, Proceedings, 3610: 456-465 2005).

The electrical models of the biological neuron were the motor which led to the design of electrical circuits, first with discrete and later with integrated components, able to generate pulses, more or less similar to biological ones. The next step was hardware implementation of processing architectures for spike-based signals, made possible by the introduction of the FPGA (field programmable gate arrays) and other programmable parallel processors. Ad hoc neuro-processors have been developed (Schoenauer, T. et al., NeuroPipe-Chip: A digital neuro-processor for spiking neural network, IEEE Transactions on Neural Networks, 13 (1) : 205-213 Jan 2002).

Spiking sensors in the form of matrices, applied to the area of vision and which code incident light intensity generating a train of pulses are described for example in Degenaar, P. et al, Adaptive ON-OFF spiking photoreceptor, Electronics Letters, 42 (4): 196-198 Feb 16 2006. This article describes a spiking vision sensor obtained by coupling an intelligent vision chip with an external circuit that generates adaptive spikes. This sensor is not therefore intrinsically spiking, in that it requires an electrical circuit to be added to the sensitive chip of the sensor. Moreover the sensor proposed in this article is not autonomous in energy terms.

In general, moreover, the possibility of controlling robots by means of spiking architectures is known; see for example Floreano, D., Evolution of spiking neural circuits in autonomous mobile robots, International Journal of Intelligent Systems, 21 (9): 1005-1024 Sept. 2006. However, to date, electrical pulses are generated by specific circuits placed downstream of the sensor, which classically offers a voltage or current output, or even simulated ones. The first element of a biomorphic control chain is not therefore present: a principle which allows the development of intrinsically spiking sensors.

Contact sensors which generate continuous signals as a result of electrical power supply and the application of forces and/or pressures on the sensitive element are known for example from EP113223, JP2002202295 and JP7140025. In particular EP113223 describes a mechanical tactile sensor for measuring the level of exerted pressure through the use of LEDs. The sensor has an empty chamber delimited by an upper flexible membrane on which the pressure is exerted. A light signal generated by a LED positioned at the rigid base of the chamber is directed to the inner surface of the membrane. A light transducer receives the signal reflected from the inner surface of the membrane and generates a current signal. As the pressure exerted changes, the geometry of the flexible membrane changes as well and, consequently, the signal intercepted by the optical transducer also changes. Through this optical process it is possible not only to measure the mechanical contact but also to measure its intensity in terms of pressure.

Other types of sensors are able to detect events of a different energy form through the generation of spike signals, such as for example in JP2049167 and US2006175931, in order to act as a trigger to maintain low levels of energy consumption. In particular US2006175931 relates to a system for detecting events of a different energy type (temperature, position, motion, impact, pressure, etc.) based on the use of a piezoelectric material as active and sensitive material. This system allows generation of high-voltage pulsed signals from the piezoelectric material.

The publications A biomimetic Haptic Sensor (Martin J. Pearson et al.) and Design, fabrication and applications of biomimetic sensors in biorobotics (P. Dario et al.) also disclose biomimetic sensors for robotic applications.

The lack of intrinsically spiking sensors can also be seen in an emerging sector with high social impact in coming years: that of cybernetic limb prostheses. One of the basic characteristics (and of the problems still only partially solved) of this family of prostheses is the transmission of sensation, or of the sensorial stimulus, to the patient via his or her peripheral nervous system. Although major progress is being made in the area of electrodes which can be implanted for connection to the peripheral nervous system, the "language" spoken by the artificial sensors currently available is not that of the biological nervous system.

Furthermore recent neurophysiological studies have suggested that in natural nervous systems the sensorial information is not coded by the firing rate of the sensors alone, but also by the spatial and time profile of the first nerve discharge from the family of sensors activated by the external stimulus. However the study and the validation of this and of other recent neurophysiological models is not to date possible due to the lack of adequate artificial sensors which can simulate natural conditions.

An object of the present invention is to provide a method for the generation of intrinsically pulse-coded sensorial information in reply to an external mechanical stimulus (force, pressure, traction, etc.) so as to imitate the sensorial information generated by a natural mechanoreceptor.

Another object of the present invention is to provide a method for the generation of such intrinsically pulse-coded sensorial information of the type mentioned above that can be used directly by the peripheral nervous system without intermediate stages of translation of the generated signal.

A further object of the present invention is to provide an artificial sensor for robotic and prosthetic applications with intrinsic pulse-coding of information, i.e. able to react to an external mechanical stimulus (force, pressure, traction, etc.) with the generation of a train of pulses, whose frequency (and its variations) codes the intensity of the same stimulus, replicating in this way the static and dynamic behaviour of a natural biological sensor.

A particular object of the present invention is to provide a biomimetic sensor of the above mentioned type which is autonomous in energy terms.

Another object of the present invention is to provide a biomimetic sensor of the above mentioned type which can be used as a sensorial element of autonomous or semiautonomous robotic systems equipped with biomimetic control architectures.

Another object of the present invention is to provide a biomimetic sensor of the above mentioned type which can be used as a sensorial element of robotic systems constituting prostheses able to be interconnected directly to the nervous system and controlled by it.

### Summary of the Invention

These objects are achieved by the method for generating intrinsically pulse-coded sensorial information according to the present invention and by the biomimetic sensor operating according to said method whose basic features are set forth in claims 1 and 8.

Other important features are disclosed in the claims dependent thereon.

### Brief description of the drawings

The features and advantages of the method for generating intrinsically pulse-coded sensorial information and of the biomimetic sensor according to the invention will be made clear from the following description of its embodiments, given as non-limiting examples with reference to the accompanying drawings in which:
Fig. 1 is an axially sectioned schematic view of a first sensor embodiment according to the invention;
Fig. 2 is an enlarged view of a detail of the sensor of Fig. 1;
Fig. 3 is an axially sectioned schematic view of a second sensor embodiment according to the invention;
Fig. 4 is a plan view from above of the sensor of Fig. 3;
Fig. 5 illustrates the flat blank of the tubular body of the sensor of Fig. 3;
Fig. 6 is an enlarged view of a detail of the sensor of Fig. 3;
Fig. 7 is an axially sectioned schematic view of a third sensor embodiment according to the invention;
Fig. 8 illustrates the flat blank of the tubular body of the sensor of Fig. 7;
Fig. 9 is an enlarged view of a detail of the sensor of Fig. 7;
Fig. 10 is an axially sectioned schematic view of a fourth sensor embodiment according to the invention;
Fig. 11 is an enlarged view of a detail of the sensor of Fig. 10;
Fig. 12 is a perspective view of the semi-tubular body defining the chamber of the sensor of Fig. 10;
Fig. 13 is a perspective view of a constructional example of the sensor according to the first embodiment;
Fig. 14 is a blown-up perspective view from above of the sensor of Fig. 13;
Fig. 15 is an exploded perspective view from below of the sensor of Fig. 13;
Fig. 16 shows the characteristic frequency curve of the signal as a function of the radius of the chamber of the sensor of Fig. 13.

### Detailed description of the invention

It is specified that in the present description the term "intrinsically biomimetic" or terms with an equivalent meaning must be understood to relate to an artificial mechanoreceptor which behaves similarly to a biological mechanoreceptor as regards the coding of the information in a pulsed mode and its transmission in the form of a train of pulses. The frequency of the pulses, and not their amplitude, is proportional to the intensity of the stimulus.

More particularly, with the term "intrinsically" it is understood that the pulsed coding is not the result of processing of the sensorial signal but instead native coding of the artificial mechanoreceptor, deriving directly from its principle of operation and its structure. Therefore biomimetism does not extend to the structure of the natural mechanoreceptor.

The term "autonomous in energy terms" or terms with equivalent meaning, referred to an artificial mechanoreceptor of the type mentioned above, must be understood as not depending for its operation on sources of energy supply external to the support system whereto it is applied, yet able to obtain directly therefrom the energy necessary for its operation.

The invention provides a method for generating a pulsed signal, of the same type as that generated by a natural mechanoreceptor inside nerve fibres when it is subjected to a mechanical stress, in a sensor which can be used for robotic and prosthetic applications when an external stimulus is applied thereon.

According to the method that forms the object of the invention, heating causes expansion of a fluid contained in an elastically deformable chamber of the sensor having an aperture with section that varies in proportion to the intensity of the force applied and a mobile shutter placed on the aperture thereof and integral with a momentum transducer. Due to expansion of the fluid contained in the chamber, the mobile shutter moves gradually away from its rest position (first position) wherein it is resting on the edge of said aperture, while between the edge of the aperture and the mobile shutter placed thereon a capillary liquid meatus is formed which, as the shutter moves away from the edge following expansion, gradually thins until it achieves a condition of capillary instability, after which it breaks.

In this condition the shutter reaches the position of maximum departure from the edge of the aperture (second position) from which, following breaking of the liquid meatus, it returns abruptly into its first position, in particular due to the action of inertial and elastic forces, reaching the edge of the aperture at non-zero speed and thus causing an impact wave which is transmitted to the transducer. The latter generates a pulse (spike) at each cycle of rising and abrupt return of the gate valve into its first position and the resulting pulse train frequency is proportional to the section of the aperture, which in turn depends on the intensity of the force applied on the sensor. In this way the signal coming out from the sensor, i.e. a pulsed signal, has a characteristic, consisting of its frequency, which can be correlated to the intensity of the stress applied to the sensor.

The dynamic that characterises the return of the shutter from the first to the second position is regulated both by the elastic properties of elastic members and by the inertial properties of inertial members both connected mechanically to the shutter. Therefore the calibration of the dynamic characteristics of the sensors (accuracy, sensitivity, frequency response) can be achieved by appropriately choosing the physical parameters of the elastic and inertial members of the sensor. This choice allows use of the sensor both in conditions of static application and in conditions of dynamic application of the external mechanical stimulus.

The thermal flow which causes expansion of the fluid contained in the chamber of the sensor is supplied by the body whereto the sensor is attached, placing this chamber in close contact with said body. More particularly this thermal flow can be supplied for example by body heat in the case of prosthetic applications or by the heat generated by electrical and/or electronic equipment in the case of applications to robotic systems with bio-inspired control architectures. In this way the sensor is able to operate without using a power supply source external to the system whereof it is part, thus being autonomous in energy terms.

The signal produced is made up of pulses which, according to the implementations, can be of the voltage, current or light intensity type. The fluid used in the chamber of the sensor can be humid and the humidity is maintained at optimal values, i.e. the sensor is maintained in a controlled atmosphere, for example placing it between two membranes, one in contact with the "warm" body and the other with the outside environment. If the aim is to make the sensor independent of the atmospheric conditions, it is possible to use different fluids, for example liquid and polymer mixtures with low vapour pressure.

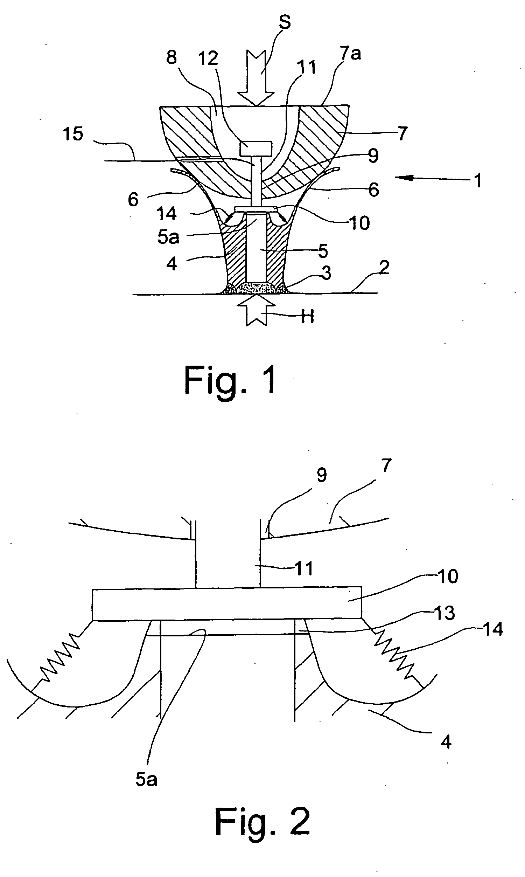

A practical implementation of the method according to the present invention is illustrated in Figures 1 and 2. Fig. 1 shows an axial section of a sensor 1 attached to a body schematically denoted as 2, for example through thermally conductive elastic contact means 3. The body 2 is able to produce a positive thermal flow towards the sensor 1 formed, for example, by the natural heat produced by the human body, in prosthetic applications, or by the heat produced by electrical and/or electronic equipment which controls and moves a robotic system with a bio-inspired control architecture.

The sensor 1 comprises a base 4 with substantially cylindrical symmetry defining a chamber 5 with a bottom wall in contact with the body 2 through the elastic contact 3 and open at the opposite side 5a in relation to said bottom wall. On the side of the base 4 where the opening 5a of the chamber 5 is formed, radially diverging, perimetrical arms 6 extend having a substantially arched shape with concavity turned outwards of the base 4. The arms 6 support a slider 7 with a substantially semi-spherical shape and a cavity 8 formed centrally on its flat section 7a. On the bottom of said cavity 8 a radial through hole 9 is formed, aligned with the symmetry axis of the base 4 turned towards the chamber 5 thereof.

On the edge of the aperture 5a of the chamber 5 a plate 10 rests and a rod-shaped piezoelectric transducer 11 extends from its centre and passes through the hole 9 up to the cavity 8 with an inertial mass 12 at its free end.

The flat section 7a of the slider 7 constitutes the interface of the sensor 1 on which an external mechanical stimulus is applied, denoted by the line S, for example a pressure force. The thermal flow, which is transmitted from the body 2 to the base 4 and to the chamber 5, is instead denoted by the line H, axially aligned and opposite the line S. As a result of the force applied on the flat section 7a of the slider 7, the latter slides towards the base 4 along the transducer 11, forcing against the arms 6 which deform elastically, diverging and inducing a corresponding radial deformation also in the base 4, which they extend from, and in the chamber 5. The widening of the chamber 5 produces an increase in the section of its aperture 5a.

Due to the effect of the heat flow H the air present in the chamber 5 expands and the pressure exerted on the plate 10 increases until the same plate rises from the edge of the aperture 5a. The rising of the plate 10 is for a certain time supported by a capillary liquid meatus 13, shown in Fig. 2, which, as the plate is raised, is thinned until it reaches a limit condition of capillary instability at which it breaks, causing the abrupt return of the plate 10 onto the edge of the aperture 5a.

According to the principles of the chemistry of surfaces, a liquid with a certain surface tension in contact with a surface condenses, filling the capillary cavities also in the presence of a gaseous environment not saturated with the vapours of the same liquid. In line with this principle, the liquid meatus 13, brought to breaking by the rising of the plate 10, will recompose, filling the capillary cavity formed between the edge of the aperture 5a and the lower surface of the same plate. According to the operating circumstances of the sensor, a liquid with suitable physical and chemical properties can be chosen as the liquid of the meatus.

Elastic return means 14 connect the plate 10 to the base 4 to aid the motion of abrupt return of the plate on the edge of the aperture 5a.

The breaking of the liquid meatus 13 allows the warm air present in the chamber 5 to exit, eliminating at the same time the thermal driving force acting on the plate 10. The discontinuance of the driving force, on the one hand, and the effect of the return force of the elastic means 14, on the other, cause the reversal of the direction of motion of the plate 10 which reaches the edge of the aperture 5a of the chamber 5 at non-zero speed, causing a mechanical impact and a consequent pulse which is transmitted to the piezoelectric transducer 11 integral with the plate 10 and generates an electric signal at the output 15 of the transducer 11. It should be pointed out that, during the phase of rising of the plate 10 and breaking of the meatus 13, the release of the warm air in the thermal chamber 5 allows simultaneous entry of cold air inside thereof before closure of the plate, therefore maintaining in equilibrium the material balance of the chamber 5.

Throughout the time of application of the force the cycle of rising and return of the plate 10 is repeated at a frequency proportional to the intensity of the applied force, as the section of the aperture 5a of the chamber 5 depends on this intensity via the cinematic chain formed by the slider 7 and the base 4 with its arms 6 and therefore the pressure acting on the plate from inside the chamber 5, responsible for the speed of movement away from the edge of the aperture 5a and the frequency of the breaking of the liquid meatus 13. In this way the sensor modulates the mechanical stimulus applied on it, generating a pulsed signal with frequency proportional to the intensity of the stimulus.

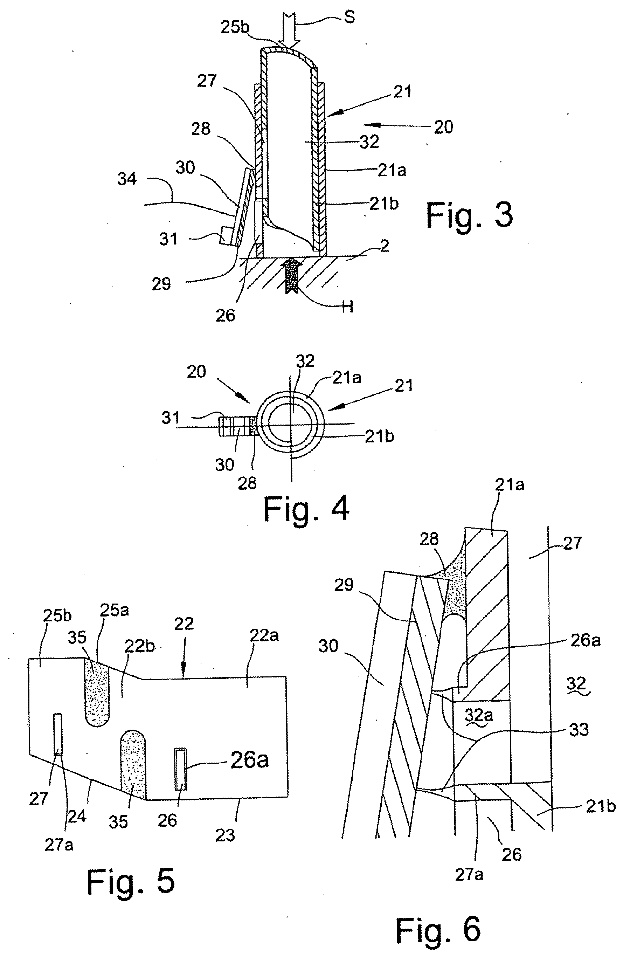

Figures 3, 4, 5 and 6 illustrate a second embodiment of the sensor according to the invention. In this case the sensor, denoted by 20, again has a geometry with substantially cylindrical symmetry yet is formed by a substantially tubular member 21 formed by a thin sheet 22 of flexible material, for example a super-elastic or shape-memory steel, whose flat blank is illustrated in Fig. 5, wound helicoidally on itself to form two coaxial coils 21a and 21b. As shown in Fig. 5, the sheet 22 is formed by two portions: a first substantially rectangular portion 22a with a base side 23 and a second portion 22b extending from one side of the portion 22a perpendicular to the base 23 and having a base side 24 extending from the base side 23 which is slanted in relation to the latter, while the opposite side is formed by a first length 25a parallel to the base side 24 and a second length 25b parallel to the base side 23. Once the sheet 22 is wound helicoidally, the first portion 22a forms the outermost coil 21a, while the portion 22b forms the innermost coil 21b. The external coil 21a is attached with its base side 23 to the warm body 2 which radiates a positive heat flow H, while the internal coil 21b projects from the external coil 21a with its lengths 25a and 25b from the opposite side in relation to the body 2 and its second length 25b is the interface with the outside of the sensor 20 on which the mechanical stimulus S is applied.

On the two portions 22a and 22b of the sheet 22 two respective windows 26 and 27 are formed in such a position that, when the sheet is wound helicoidally on itself to form the two coils 21a and 21b, the windows are situated one corresponding to the other. The two windows 26 and 27 are also at two different levels in relation to the base side 23 of the portion 22a of the sheet 22, so that, when they correspond one to the other, they define a section with passage smaller than, or at most equal to, the section of each of them.

A plate 29 with shape corresponding to that of the window 26 formed on the portion 22a is hinged, through elastic return means 28, to the external coil 21a at the window 26 with one of its sides and a piezoelectric transducer 30 and an inertial mass 31 are placed thereon.

In particular, the two windows 26 and 27 are rectangular in shape and the window 26 of the external coil 21a has a projecting perimeter edge 26a extending along three of its sides, while the window 27 of the internal coil 21b has an edge 27a projecting along the remaining side and extending externally as far as the same level of the edge 26a, the edges 26a and 27a being coplanar and forming the boundary edge of an aperture 32a of a chamber 32 delimited by two coils 21a and 21b wound one on the other.

As a result of the shape of the sheet 22 and its flexibility, when a stimulus S, for example a pressure force, is applied on the second length 25b of the internal coil 21b, the latter slides within the external coil 21a, thus varying, in particular increasing, the section of the aperture 32a. Through the effect of the heat flow H the air contained in the chamber 32 expands, causing repeated cycles of gradual movement away and abrupt return of the plate 29 on the edge of the aperture 32a whenever the capillary liquid meatus 33 formed between the plate 29 and the edge of the aperture 32a breaks. The result in this case too is the generation of a train of pulses by the piezoelectric transducer 30 at its output 34 with frequency proportional to the intensity of the applied force.

Advantageously, in order to increase the flexibility of the sheet 22 and reduce its elastic shear rigidity, on its portion 22b discharge cuts 35 can be provided, as shown in Fig. 5.

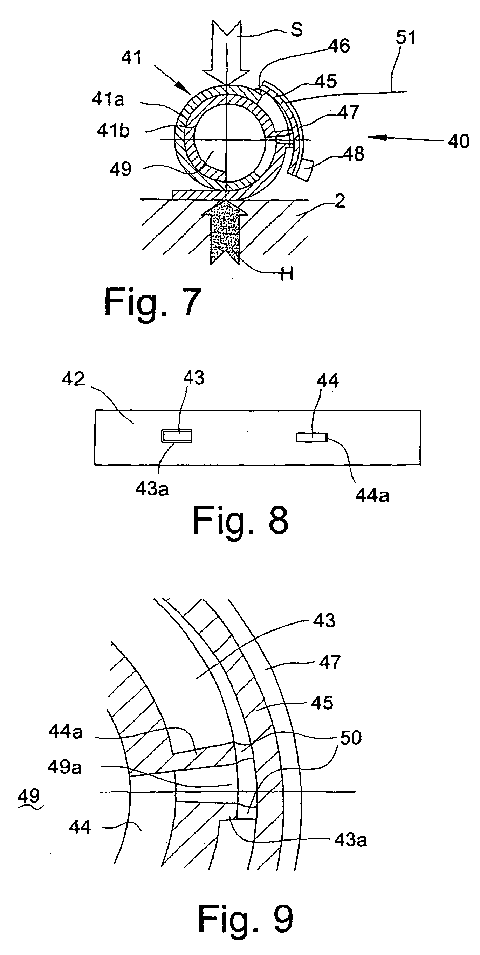

A third embodiment of the sensor according to the invention is illustrated in Figures 7, 8 and 9. In this case the sensor, denoted overall by 40, has a configuration similar to that of the second embodiment. As a matter of fact, a substantially tubular member 41 is found again, formed by a thin sheet 42 of flexible material, for example a super-elastic or shape-memory steel, whose flat blank is shown in Fig. 8, wound helicoidally on itself to form two coaxial coils 41a and 41b, external and internal coils respectively. However in this case the application of the stimulus S and the transmission of the thermal energy H from the body 2 which the sensor 40 is attached to through its external coil 41a attached thereto, take place radially instead of axially. The different application of the stimulus causes the reciprocal circumferential sliding between the two coils 41a and 41b and the reduction of the radial dimension of the tubular member 41.

Two windows 43 and 44 are formed on the sheet 42, aligned and spaced one in relation to the other in order to correspond at least partially when the sheet 42 is wound helicoidally in two coaxial coils. In this case the two windows extend circumferentially along the two coils and a plate 45, also with a circumferential extension, extends on the window 43 formed on the external coil 41a and is hinged, through elastic return means 46, with one of its sides, to the external coil 41a at the window 43 and a piezoelectric transducer 47 and an inertial mass 48 are arranged on it.

As in the second embodiment, the plate 45 rests on the edge of an aperture 49a of a chamber 49 defined by the sheet 42 wound in two coaxial coils, said edge being formed by raised edges 43a and 44a of the two windows 43 and 44, as in the previous embodiment. A capillary liquid meatus 50 is formed between the plate 45 and the edge of the aperture 49a.

The mutual circumferential sliding between the two coils 41a and 41b causes a variation, in particular an increase, in the section of the aperture 49a, which allows, in this case too, a correlation to be established between the intensity of the force applied radially on the tubular member 41, which generates the variation in section of the aperture 49a, and the frequency of the train of pulses generated by the cyclical gradual movement away and rapid return of the plate 45 against the edge of the aperture 49a whenever the capillary liquid meatus 50 breaks through the effect of reaching the limit of capillary instability following expansion of the air contained in the chamber 49 as a result of the heat flow H.

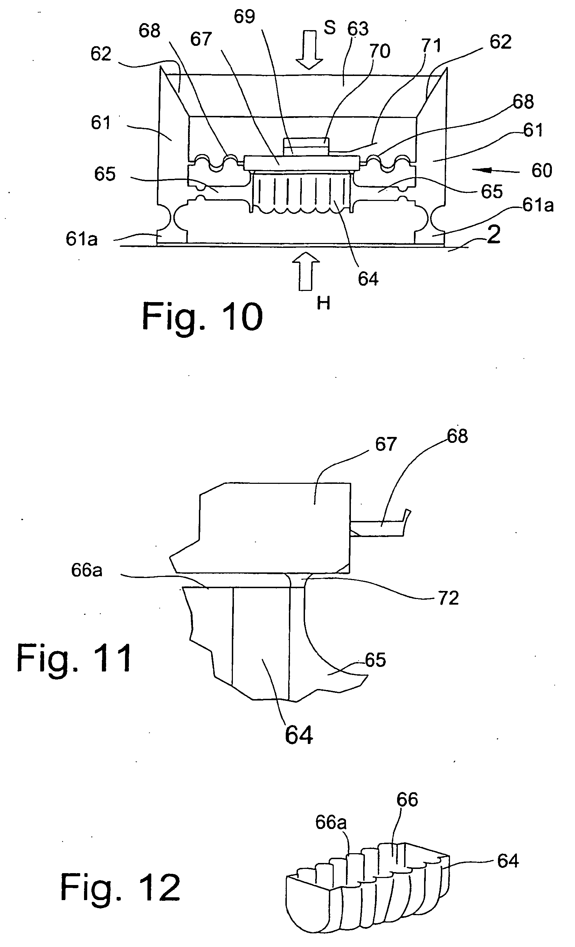

A fourth embodiment of the sensor according to the invention is shown in Figures 10, 11 and 12. In this case the sensor, denoted overall by 60, has a conceptually similar configuration to that of the first embodiment, illustrated above, yet has a linear development instead of one with cylindrical symmetry. This configuration is particularly suitable for a technological application of micromanufacture.

The sensor 60 is substantially formed by two walls or uprights 61, parallel and spaced apart, attached with their bases 61a to the body 2, suitable for transmitting to the sensor a positive heat flow H. The free ends of the two uprights 61 have respective faces 62 equally slanted and turned one towards the other which support a wedge slider 63 formed by a body with truncated pyramid section with slanting sides which abut on the correspondingly slanted faces 62 of the uprights 61. The body 63 forms the interface with the outside of the sensor 60 and the mechanical stimulus denoted by S is applied thereon.

Between the two uprights 61 a substantially semi-tubular body 64 is placed with axis orthogonal to the same uprights, closed at the ends and supported through them by two aligned arms 65 extending from the uprights 61. The semi-tubular body 64 is made in axially elastic material, being shaped for example substantially as bellows, and defines in its interior a chamber 66 having an aperture 66a turned towards the slider 63 on the edge of which a plate 67 rests, also connected laterally to the uprights 61 via elastic means 68. A piezoelectric transducer 69 and an inertial mass 70 are integral with the plate 67. The transducer comprises an output 71 for the transmission of the generated signal.

As in the previous embodiments, the fluid contained in the chamber 66 expands through the effect of the heat flow H. At the same time, through the effect of the stimulus S, for example a pressure force, the slider 63 slides along the slanted faces 62 of the uprights 61 causing their bending outwards. The bending of the uprights 61 is transmitted to the semi-tubular body 64 in the form of traction exerted by the arms 65 with consequent axial expansion of the body 64 and a change in the section of the aperture 66a of the chamber 66, on whose edge the plate 67 rests. As a result of the expansion of the fluid contained therein through the effect of the heat flow H, the pressure inside the chamber 66, causes the gradual movement away of the plate 67 from the body 64 supported by the capillary liquid meatus 72 which is formed between the same plate and the edge of the aperture 66a, until reaching the limit of capillary instability, with identical operation of the sensor to that described for the previous cases.

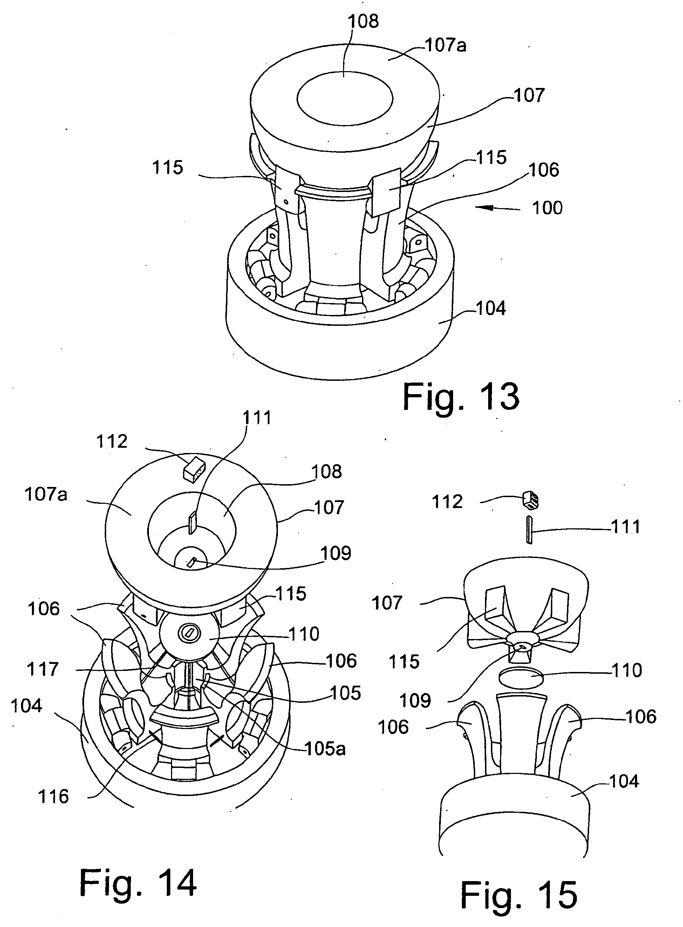

Figures 13, 14 and 15 show a construction example of the sensor according to the first embodiment of the invention shown in Figures 1 and 2. The sensor 100 illustrated in Figures 13-15 comprises a base 104 with substantially cylindrical symmetry defining a chamber 105 with a thermally conductive closed base, open on the opposite side 105a in relation to said base. The base 104 is intended to be placed in contact with a body at such a temperature as to ensure a positive heat flow towards the sensor. On the side of the base 104 where the aperture 105a of the chamber 105 is formed, radially diverging perimeter arms 106 extend, with a substantially arched shape with concavity turned towards the exterior of the base 104. A slider 107 rests on the arms 106, the slider having a substantially semi-spherical shape and being formed with a central cavity 108 on its flat section 107a. On the base of said cavity 108 a radial through hole 109 is formed, aligned with the axis of symmetry of the base 104 turned towards the chamber 105 thereof.

The slider 107 has a plurality of angularly spaced outer ridges 115, two by two forming guide members between which a respective arm 106 is placed.

A plate 110 rests on the edge of the aperture 105a of the chamber 105 and a rod-shaped piezoelectric transducer 111 extends from the centre of the plate, passing through the hole 109 and supporting an inertial mass 112 at its end in the chamber 108.

Elastic means 116 are provided between the single pairs of arms 106 to ensure the tightness of the chamber 105 and an adequate elastic return towards the condition of rest of the arms 106, whereas further elastic means 117 are provided between the plate 110 and the arms 106 to obtain an adequate elastic return of the plate 110 on the edge of the aperture 105a.

The flat section 107a of the slider 107 constitutes the interface of the sensor 100 on which an external mechanical stimulus is applied, for example a pressure force. As a result of the force applied on the flat section 107a of the slider 107, the latter slides towards the base 104 along the transducer 111 forcing against the arms 106 which deform elastically, inducing a corresponding radial deformation also in the base 104, from which they extend, and in the chamber 105. The widening of the chamber 105 produces an increase in the section of its aperture 105a.

Through the effect of the heat flow the air present in the chamber 105 expands and the pressure exerted on the plate 110 increases until the same plate rises from the edge of the aperture 105a. The rising of the plate 110 is for a certain time supported by a capillary liquid meatus (not shown) which, as the plate rises, thins until it reaches a limit condition of capillary instability at which it breaks, causing the abrupt return of the plate 110 on the edge of the aperture 105a. The breaking of the liquid meatus allows the warm air present in the chamber 105 to exit, at the same time eliminating the thermal driving force acting on the plate. The discontinuance of the driving force, on the one hand, and the effect of the return force of the elastic means 117, on the other one, cause reversal of the motion of the plate 110 which reaches the edge of the aperture 105a of the chamber 105 at non-zero speed, causing mechanical impact and a consequent pulse which, transmitting to the piezoelectric transducer 111 integral with the plate 110, produces an electric output signal from the transducer 111.

Throughout the time of application of the force the cycle of rising and return of the plate 110 is repeated with a frequency proportional to the intensity of the applied force, because the section of the aperture 105a of the chamber 105 depends on this intensity through the cinematic chain formed by the slider 107 and the base 104 with its arms 106 and therefore the force acting on the plate from the interior of the chamber 105, responsible for the speed of movement away from the edge of the aperture 105a and the frequency of breaking of the liquid meatus. In this way the sensor modulates the mechanical stimulus applied thereon, generating a pulsed signal with frequency proportional to the intensity of the stimulus.

Fig. 16 shows the trend of the characteristic frequency of the pulsed signal in relation to a characteristic parameter, i.e. the radius of the heating chamber. This diagram clearly shows that as the force applied on the slider increases, and hence as the radius of the chamber increases, the frequency of the pulses increases, in this way causing the frequency coding of electrical pulses of the continuous source signal originating from a mechanical stimulus.

These results are comparable with the frequencies involved in biological signals.

By way of information, Merkel's disk, one of the mechanoreceptors of the human skin, responds to static stimuli with trains of pulses whose frequency rises linearly with the intensity of the stimulus (which results in skin indentation). In particular, for indentations of the skin between 50 and 300 µ, the firing rate rises from 40 to 200 pulses per second.

The Pacinian corpuscle reaches much higher spiking rates. It is another of the mechanoreceptors of the human skin, more sensitive to dynamic stimuli of the vibrational type: it has a typical length of 1 mm and its firing rate varies from 50 to more than 800 Hz, with a peak of sensitivity of around 250-300 Hz: in other words, this mechanoreceptor is particularly sensitive to tactile stimuli oscillating at a frequency of approximately 250-300 Hz which are coded with trains of pulses at this specific frequency (each oscillation is coded with a pulse).

All the biological sensors produce pulsed electrical signals which code the external stimulus: in other words the language used by all the animal nervous systems is made up exclusively of pulses or *spikes,* whose amplitude is approximately 80 mV and whose frequency is proportional, *inter alia,* to the intensity of the stimulus. For the mechanism with which these action potentials are generated, an action potential is immediately followed by a refractory period lasting 0.8-1.0 ms, during which another spike cannot be generated (due to the disactivation of the Na+ channels). The upper limit of the firing rate in biological systems is 1000-1200 spikes per second. For example the number of action potentials per second generated by a family of mechanoreceptors with slow adaptation of the human tactile system varies linearly with the indentation of the skin caused by the external stimulus, rising by 10 pulses per second for every 200 µ of indentation (with a minimum indentation detected by the sensor of 80 p). That is to say, with an indentation of 1 mm, the firing rate is 40 Hz, while at 1.4 mm it rises to 60 Hz.

In the same way, from a careful analysis of the characteristic curve shown in Fig. 16, a variation is noted of 10 Hz in the firing rate (from approximately 273 Hz to approximately 283 Hz), changing from a radius of the thermal chamber of 0.3 mm to a value of 0.5 mm. Therefore, by appropriately designing the transmission member which transfers the external mechanical signal (stimulus) in terms of deformation of the thermal chamber, it is possible to establish a linear relationship between the axial indentation generated by the external stimulus and the radial deformation of the sensor, thus obtaining a law of variation of frequency comparable to the natural one (50 Hz/mm).

From what is stated above it is clear how, through the method and the sensor according to the invention, it is possible to indicate the presence of force/pressure applied on the sensor through the generation of spikes or electric pulses. These spikes are generated at a frequency which is directly proportional to the force/pressure applied. In this way the sensor codes the force/pressure signal through trains of electric pulses in the same way in which the natural biological mechanoreceptors generate electrical pulses inside nerve fibres when subjected to mechanical stresses.

The main advantages of the present invention are: intrinsically biomimetic operation of the sensor, energy self-sufficiency of the sensor and the reduced need for electrical connections - only two electrical wires or a single optical fibre transmit the signal generated by the sensor, while no external power supply wire is required.

Additional advantages which can be achieved thanks to the invention relate to:
- the possibility of building autonomous or semi-autonomous robotic systems with biomimetic control architectures, overcoming the limit due to the use of electronic equipment to convert the continuous signal coming from a non-intrinsically biomimetic sensor into a pulsed signal replicating a realistic biological signal;
- the possibility of directly interconnecting robotic systems constituting prostheses to the nervous system without the need to provide the prosthesis with electronic equipment able to code the signal coming from the artificial sensors with which the prosthesis is provided into biomimetic signals to be sent to the biological nervous system;
- the possibility of performing neurosensorial research with a high scientific content, solving the ethical problem of the use in clinical practice of sensors taken from animal models;
- the possibility of performing the mechanical characterisation of any type of material using a biomorphic recognition of the different elastic properties, thus avoiding the use of complicated theoretical models and experimental apparatus for the purposes of a quantitative assessment of these properties.

Variations and/or changes can be made to the method for generating intrinsically spiked sensorial information and to the biomimetic sensor for robotic and prosthetic applications according to the invention without departing from the scope of the invention as defined in the following claims.

## Claims

1. Method for generating intrinsically pulse-coded sensorial information for robotic and prosthetic applications, as a result of an external mechanical stimulus (S) exerted on a sensor (1, 20, 40, 60, 100) applied to a body (2) suitable for emitting a positive heat flow (H) to said sensor, **characterised in that** it comprises the following steps:
- causing of a change in section of an aperture (5a, 32a, 49a, 66a, 105a) of a chamber (5, 32, 49, 66, 105) of said sensor proportional to the intensity of said stimulus, said chamber containing an expandable moist fluid, a mobile member (10, 29, 45, 67, 110) being placed on said aperture which temporarily closes said chamber;
- transmitting to said mobile member a repeated reciprocal movement between a first position, in which it is in contact with the edge of said aperture, and a second position, corresponding to a condition of capillary instability of a liquid meatus (13, 33, 50, 72) which grows gradually between said mobile member and the edge of said aperture while said mobile member moves towards said second position, said movement being produced by the expansion of the fluid contained in said chamber and by the consequent increase in pressure in it caused by the heat flow transmitted by said body to said sensor, due to the condition of capillary instability reached in said second position said meatus breaking and causing the abrupt return of said mobile member into said first position, in this way closing again said chamber and restoring the condition for a new increase in pressure in it;
- converting of the momentum exchanged at each impact between said mobile member and the edge of said aperture to a corresponding pulse and generating, throughout the duration of said external mechanical stimulus, a train of pulses of frequency proportional to the section of the aperture of said chamber and therefore to the intensity of said stimulus.

2. Method for generating intrinsically pulse-coded sensorial information according to claim 1, wherein said pulse is a voltage, current or light intensity pulse.

3. Method for generating intrinsically pulse-coded sensorial information according to claims 1 or 2, wherein said pulse is an electrical pulse generated by a piezoelectric member (11, 30, 47, 69, 111) associated with said mobile member.

4. Method for generating intrinsically pulse-coded sensorial information according to any one of the previous claims, wherein said positive heat flow is constituted by the natural heat of the human body.

5. Method for generating intrinsically pulse-coded sensorial information according to any one of claims 1, 2 or 3, wherein said positive heat flow is constituted by the heat generated by electrical and/or electronic equipment.

6. Method for generating intrinsically pulse-coded sensorial information according to any one of the previous claims, wherein the change in section of the aperture of said chamber is produced by the sliding of a slider (7, 63, 107) on which said external mechanical stimulus (S) acts, said sliding causing the elastic deformation of said chamber.

7. Method for generating intrinsically pulse-coded sensorial information according to any one of claims 1 to 5, wherein the change in section of the aperture of said chamber is caused by an elastically reversible relative sliding of walls (21b, 41b) defining said chamber produced by said external mechanical stimulus.

8. Biomimetic sensor for robotic and prosthetic applications, **characterised in that** it comprises an elastically deformable chamber (5, 32, 49, 66, 105) with an aperture (5a, 32a, 49a, 66a, 105a) whose section varies as a result of deformation of said chamber and containing a moist fluid which can be expanded through the effect of a positive heat flow (H) transmitted by a body (2) whereto said sensor is applied, a mobile member (10, 29, 45, 67, 110) placed on said aperture and suitable for moving, reciprocally and repeatedly, between a first position, in which it is in contact with the edge of said aperture, and a second position, where it is spaced apart therefrom, transducer means (11, 30, 47, 69, 111) connected to said mobile member and suitable for generating a train of pulses in response to corresponding impacts of said mobile member against the edge of said aperture at the end of the return travel into said first position, a mobile interface (7, 21b, 41a, 63, 107) of said chamber on which an external mechanical stimulus (S) acts whose intensity is proportional to the variation of the section of the aperture of said chamber, the expansion of said fluid producing an increase in pressure in said chamber which moves said mobile member from said first position to said second position, during this movement said mobile member being supported by a capillary liquid meatus (13, 33, 50, 72) which grows gradually between it and the edge of said aperture until reaching a condition of capillary instability at said second position, at which said meatus breaks, causing the abrupt return of said mobile member into its first position, in this way closing again said chamber and restoring the condition for a new increase of pressure in it, the frequency of the train of pulses thus generated being proportional to the section of said aperture and therefore to the intensity of the external mechanical stimulus acting on said interface.

9. Biomimetic sensor according to claim 8, wherein said chamber (5, 105) is formed in a body (4, 104) of a sensor with substantially cylindrical symmetry, said aperture (5a, 105a) being formed at one of its ends, and said interface being formed by a slider moving along the axis of said chamber connected slidingly to said sensor body in such a way that one of its movements towards said sensor body produces a change in section of said aperture.

10. Biomimetic sensor according to claim 9, wherein said sensor body (4, 104) has a crown of divergent arms (6, 106) extending from its end on which said aperture is formed, said slider (7, 107) being supported by said arms slidingly between them.

11. Biomimetic sensor according to any one of claims 8 to 10, wherein said transducer means comprise a piezoelectric member (11, 111) extending from said mobile member (10, 110).

12. Biomimetic sensor according to claim 11, wherein a cavity (8, 108) is formed on said slider (7, 107), said piezoelectric member (11, 111) extending in it and constituting the member for guiding the sliding of said slider.

13. Biomimetic sensor according to any one of claims 9 to 12, wherein said slider (107) has a substantially semi-spherical shape, equidistant ridges (115) being formed on its external surface between which said diverging arms (106) are arranged.

14. Biomimetic sensor according to any one of claims 9 to 13, wherein said diverging arms (6, 106) are elastically connected one another.

15. Biomimetic sensor according to claim 8, wherein said chamber is formed in a sensor body (21, 41) (22, 42) formed by a metal sheet bent into two concentric coils (21a, 21b, 41a, 41b) on which respective corresponding windows (26, 27, 43, 44) are formed so as to form an aperture (32a, 49a) of said chamber (32, 49) with section that varies following relative sliding of said two coils one in relation to the other caused by the external mechanical stimulus acting on said mobile interface, said mobile member being formed by a plate hinged to the edge of the window formed on the external coil.

16. Biomimetic sensor according to claim 15, wherein said body (21) of a sensor (20) is attached orthogonally to said body (2) with an axial end of the external coil (21b) and said mobile interface (25b) is constituted by the free edge of the opposite axial end of the internal coil (21b), the other end of said internal coil being spaced apart from said body (2) to allow said relative sliding, said windows (26, 27) being oriented axially on said coils.

17. Biomimetic sensor according to claim 15, wherein said body (41) of a sensor (40) is in contact with said body (2) along one of its generatrices, said mobile interface being formed substantially by the diametrically opposed generatrix, said windows (43, 44) being oriented circumferentially on said coils (41a, 41b).

18. Biomimetic sensor according to claim 8, wherein said chamber (66) is formed in a substantially semitubular body (64) of a sensor (60) which can be deformed elastically in an axial direction and connected rigidly with its ends to a pair of parallel and flexible supports (61), said mobile member being a plate (67) placed on a diametrical aperture (66a) of said sensor body (64), said mobile interface being a wedge slider (63) engaged slidingly between said supports (61), whereby a movement of said slider towards said sensor body caused by said external mechanical stimulus (S) causes the spreading apart of said supports and an axial elongation of said sensor body (64) with consequent variation in the section of its diametrical aperture (66a).

19. Biomimetic sensor according to any one of claims 15 to 18, wherein said transducer means comprise a piezoelectric member (30, 47, 69) connected to said mobile member (29, 45, 67).

20. Biomimetic sensor according to any one of the previous claims, wherein a replaceable inertial mass (12, 31, 48, 70, 112) is associated with said mobile member.

21. Biomimetic sensor according to any one of the previous claims, wherein elastic return means (14, 28, 46, 68, 117) are provided between said mobile member and said sensor body.

## Patentansprüche

1. Verfahren zum Erzeugen von intrinsisch impulskodierten sensorischen Informationen für robotische und prothetische Anwendungen als ein Ergebnis einer externen mechanischen Stimulation (S), die auf einen Sensor (1, 20, 40, 60, 100) ausgeübt wird, der an einem Körper (2) angewandt wird, der geeignet ist, um eine positive Wärmeströmung (H) zu dem Sensor zu emittieren, **dadurch gekennzeichnet, dass** es die folgenden Schritte enthält:
- Veranlassen einer Änderung im Querschnitt einer Öffnung (5a, 32a, 49a, 66a, 105a) einer Kammer (5, 32, 49, 66, 105) des Sensors proportional zu der Intensität der Stimulation, welche Kammer ein ausdehnbares feuchtes Fluid enthält, wobei auf der Öffnung ein mobiles Element (10, 29, 45, 67, 110) platziert wird, das die Kammer temporär verschließt;
- Übertragen einer wiederholt reziproken Bewegung auf das mobile Element zwischen einer ersten Position, in der es in Kontakt mit dem Rand der Öffnung ist, und einer zweiten Position, die einem Zustand einer kapillaren Instabilität eines Flüssigkeitskanals (13, 33, 50, 72) entspricht, der graduell zwischen dem mobilen Element und dem Rand der Öffnung zunimmt, während sich das mobile Element zu der zweiten Position hin bewegt, welche Bewegung durch die Expansion des Fluids, das in der Kammer enthalten ist, und durch die konsequente Druckzunahme darin erzeugt wird, die durch die Wärmeströmung verursacht wird, die von dem Körper zu dem Sensor übertragen wird, wobei auf Grund des Zustands der kapillaren Instabilität, die in der zweiten Position erreicht wird, der Kanal bricht und die abrupte Rückkehr des mobilen Elementes in die erste Position verursacht, auf welche Weise es die Kammer wieder verschließt und den Zustand für eine neue Druckzunahme darin wieder herstellt;
- Konvertieren des Moments, das bei jedem Stoß zwischen dem mobilen Element und dem Rand der Öffnung ausgetauscht wird, in einen entsprechenden Impuls und Erzeugen eines Impulszuges mit einer Frequenz proportional zu dem Querschnitt der Öffnung der Kammer und daher zu der Intensität der Stimmulation während der Dauer der externen mechanischen Stimmulation.

2. Verfahren zum Erzeugen von intrinsisch impulskodierten sensorischen Informationen nach Anspruch 1, wobei der Impuls ein Spannungs-, Strom- oder Lichtintensitätsimpuls ist.

3. Verfahren zum Erzeugen von intrinsisch impulskodierten sensorischen Informationen nach Anspruch 1 oder 2, wobei der Impuls ein elektrischer Impuls ist, der durch ein piezoelektrisches Element (11, 30, 47, 69, 111) erzeugt wird, das mit dem mobilen Element verbunden ist.

4. Verfahren zum Erzeugen von intrinsisch impulskodierten sensorischen Informationen nach einem der vorhergehenden Ansprüche, wobei die positive Wärmeströmung durch die natürliche Wärme des menschlichen Körpers gebildet wird.

5. Verfahren zum Erzeugen von intrinsisch impulskodierten sensorischen Informationen nach einem der Ansprüche 1, 2 oder 3, wobei die positive Wärmeströmung durch die Wärme gebildet wird, die durch elektrisches und/oder elektronisches Equipment erzeugt wird.

6. Verfahren zum Erzeugen von intrinsisch impulskodierten sensorischen Informationen nach einem der vorhergehenden Ansprüche, wobei die Änderung im Querschnitt der Öffnung der Kammer durch das Verschieben eines Schiebers (7, 63, 107) erzeugt wird, auf welchen die externe mechanische Stimmulation (S) wirkt, welches Verschieben die elastische Deformation der Kammer verursacht.

7. Verfahren zum Erzeugen von intrinsisch impulskodierten sensorischen Informationen nach einem der Ansprüche 1 bis 5, wobei die Änderung im Querschnitt der Öffnung der Kammer durch ein elastisch reversibles relatives Verschieben von Wänden (21b, 41b) verursacht wird, die die Kammer definieren, was durch die externe mechanische Stimmulation erzeugt wird.

8. Biomimetrischer Sensor für robische und prothetische Anwendungen, **dadurch gekennzeichnet, dass** er enthält eine elastisch deformierbare Kammer (5, 32, 49, 66, 105) mit einer Öffnung (5a, 32a, 49a, 66a, 105a), deren Querschnitt als eine Folge einer Deformation der Kammer variiert, und enthaltend ein feuchtes Fluid, das durch die Wirkung einer positiven Wärmeströmung (H) expandieren kann, der durch einen Körper (2) übertragen wird, auf den der Sensor angewandt wird, ein mobiles Element (10, 29, 45, 67, 110), das an der Öffnung platziert ist und geeignet ist, um sich zwischen einer ersten Position, in welcher es in Kontakt mit dem Rand der Öffnung ist, und einer zweiten Position, wo es davon beabstandet ist, reziprok und wiederholt zu bewegen, Wandlereinrichtungen (11, 30, 47, 69, 111) die mit dem mobilen Element verbunden sind und geeignet sind, um einen Impulszug in Abhängigkeit von entsprechenden Stößen des mobilen Elemements gegen den Rand der Öffnung am Ende der Rückkehrbewegung in die erste Position zu erzeugen, ein mobiles Interface (7, 21b, 41a, 63, 107) der Kammer, worauf eine externe mechanische Stimmulation (S) wirkt, deren Intensität proportional zu der Variation des Querschnittes der Öffnung der Kammer ist, wobei die Expansion des Fluids eine Druckzunahme in der Kammer erzeugt, was das mobile Element aus der ersten Position zu der zweiten Position bewegt, wobei während dieser Bewegung das mobile Element von einem kapillaren Flüssigkeitskanal (13, 33, 50, 72) getragen wird, der graduell zwischen ihm und dem Rand der Öffnung zunimmt, bis ein Zustand einer kapillaren Instabilität an der zweiten Position erreicht wird, bei dem der Kanal bricht, was die abrupte Rückkehr des mobilen Elements in seine erste Position verursacht, wobei auf diese Weise die Kammer wieder geschlossen wird und der Zustand für eine neue Druckzunahme darin wieder hergestellt wird, wobei die Frequenz des somit erzeugten Impulszuges proportional zu dem Querschnitt der Öffnung und daher zu der Intensität der externen mechanischen Stimmulation ist, die auf das Interface wirkt.

9. Biomimetrischer Sensor nach Anspruch 8, wobei die Kammer (5, 105) in einem Körper (4, 104) eines Sensors mit in wesentlichen zylindrischer Symmetrie gebildet ist, wobei die Öffnung (5a, 105a) an einem ihrer Enden ausgebildet ist, und das Interface durch einen sich längs der Achse der Kammer bewegenden Schieber gebildet ist, der in solcher Weise verschiebbar mit dem Sensorkörper verbunden ist, dass eine seiner Bewegungen zu dem Sensorkörper eine Änderung im Querschnitt der Öffnung erzeugt.

10. Biomimetrischer Sensor nach Anspruch 9, wobei der Sensorkörper (4, 104) eine Krone von divergenten Armen (6, 106) hat, die sich von seinem Ende, an dem die Öffnung ausgebildet ist, erstrecken, wobei der Schieber (7, 107) von den Armen zwischen ihnen verschiebbar gehalten ist.

11. Biomimetrischer Sensor nach einem der Ansprüche 8 bis 10, wobei die Wandlereinrichtungen ein piezoelektrisches Element (11, 111) enthalten, das sich von dem mobilen Element (10, 110) aus erstreckt.

12. Biomimetrischer Sensor nach Anspruch 11, wobei eine Kavität (8, 108) an dem Schieber (7, 107) ausgebildet ist, wobei sich das piezoelektrische Element (11, 111) darin erstreckt und das Element zum Führen des Verschiebens des Schiebers bildet.

13. Biomimetrischer Sensor nach einem der Ansprüche 9 bis 12, wobei der Schieber (107) eine im wesentlichen halbkugelförmige Form hat, wobei äquidistante Rippen (115) an seiner äußeren Oberfläche ausgebildet sind, zwischen welchen die divergierenden Arme (106) angeordnet sind.

14. Biomimetrischer Sensor nach einem der Ansprüche 9 bis 13, wobei die divergierenden Arme (6, 106) elastisch miteinander verbunden sind.

15. Biomimetrischer Sensor nach Anspruch 8, wobei die Kammer in einem Sensorkörper (21, 41) (22, 42) gebildet ist, der durch ein Metallblech gebildet ist, das in zwei konzentrische Wicklungen (21a, 21b, 41a, 41b) gebogen ist, woran jeweils entsprechende Fenster (26, 27, 43, 44) ausgebildet sind, um eine Öffnung (32a, 49a) der Kammer (32, 49) mit einem Querschnitt zu bilden, der einem relativen Verschieben der zwei Wicklungen von einer in Relation zur anderen folgend variiert, verursacht durch die externe mechanische Stimmulation, die auf das mobile Interface wirkt, wobei das mobile Element durch eine Platte gebildet ist, die am Rand des Fensters angelenkt ist, das an der äußeren Wicklung ausgebildet ist.

16. Biomimetrischer Sensor nach Anspruch 15, wobei der Körper (21) eines Sensors (20) orthogonal zu dem Körper (2) angebracht ist, wobei ein axiales Ende der externen Wicklung (21b) und das mobile Interface (25b) durch den freien Rand des entgegengesetzten axialen Endes der inneren Wicklung (21b) gebildet sind, wobei das andere Ende der internen Wicklung von dem Körper (2) beabstandet ist, um das relative Verschieben zu gestatten, wobei die Fenster (26, 27) axial an den Wicklungen orientiert sind.

17. Biomimetrischer Sensor nach Anspruch 15, wobei der Körper (41) eines Sensors (40) in Kontakt mit dem Körper (2) längs einer seiner Erzeugenden ist, wobei das mobile Interface im wesentlichen durch die diametral entgegengesetzte Erzeugende gebildet ist, wobei die Fenster (43, 44) umfangsmäßig an den Wicklungen (41a, 41b) orientiert sind.

18. Biomimetrischer Sensor nach Anspruch 8, wobei die Kammer (66) in einem im wesentlichen halbröhrenartigen Körper (64) eines Sensors (60) gebildet ist, der elastisch in einer Axialrichtung deformiert werden kann und starr mit seinen Enden mit einem Paar von parallelen und flexiblen Haltern (61) verbunden ist, wobei das mobile Element eine Platte (67) ist, die an einer diametralen Öffnung (66a) des Sensorkörpers (64) platziert ist, wobei das mobile Interface ein Keilschieber (63) ist, der verschiebbar zwischen Haltern (61) in Eingriff ist, wodurch eine Bewegung des Schiebers zu dem Sensorkörper, verursacht durch die externe mechanische Stimmulation (S) das Auseinanderspreizen der Halter und eine axiale Verlängerung des Sensorkörpers (64) mit konsequenter Variation im Querschnitt seiner diametralen Öffnung (66a) verursacht.

19. Biomimetrischer Sensor nach einem der Ansprüche 15 bis 18, wobei die Wandlereinrichtungen ein piezoelektrisches Element (30, 47, 69) enthalten, das mit dem mobilen Element (29, 45, 67) verbunden ist.

20. Biomimetrischer Sensor nach einem der vorhergehenden Ansprüche, wobei eine austauschbare Inertialmasse (12, 31, 48, 70, 112) mit dem mobilen Element verbunden ist.

21. Biomimetrischer Sensor nach einem der vorhergehenden Ansprüche, wobei elastische Rückkehreinrichtungen (14, 28, 46, 68, 117) zwischen dem mobilen Element und dem Sensorkörper vorgesehen sind.

## Revendications

1. Procédé pour générer des informations sensorielles intrinsèquement codées par impulsions pour des applications robotiques et prothétiques, suite à un stimulus mécanique externe (S) appliqué sur un capteur (1, 20, 40, 60, 100) situé sur un corps (2) apte à émettre vers ledit capteur un flux de chaleur positive (H), **caractérisé en ce qu'**il comprend les étapes suivantes :
- provoquer un changement de section d'une ouverture (5a, 32a, 49a, 66a, 105a) d'une chambre (5, 32, 49, 66, 105) du dit capteur proportionnellement à l'intensité du dit stimulus, ladite chambre contenant un fluide humide expansible, un élément mobile (10, 29, 45, 67, 110) situé sur ladite ouverture obturant temporairement ladite chambre ;
- transmettre au dit élément mobile un mouvement alternatif répété entre une première position, dans laquelle il est en contact avec le bord de ladite ouverture, et une seconde position, correspondant à un état d'instabilité capillaire d'un méat liquide (13, 33, 50, 72) qui croît graduellement entre ledit élément mobile et le bord de ladite ouverture lorsque ledit élément mobile se déplace vers ladite seconde position, ledit mouvement étant produit par l'expansion du fluide contenu dans ladite chambre et par augmentation conséquente de la pression dans celle-ci causée par le flux de chaleur transmis par ledit corps au dit capteur, à cause de l'état d'instabilité capillaire atteint dans ladite seconde position, ledit méat se rompant et provoquant le brusque retour du dit élément mobile dans la première position, fermant ainsi à nouveau ladite chambre et restaurant les conditions pour une nouvelle augmentation de pression dans celle-ci ;
- convertir la quantité de mouvement échangée lors de chaque impact entre ledit élément mobile et le bord de ladite ouverture en une impulsion correspondante et générer, pendant toute la durée du dit stimulus mécanique externe, un train d'impulsions de fréquence proportionnelle à la section de l'ouverture de ladite chambre et donc à l'intensité du dit stimulus.

2. Procédé pour générer des informations sensorielles intrinsèquement codées par impulsions selon la revendication 1, où ladite impulsion est une impulsion de tension, de courant ou d'intensité lumineuse.

3. Procédé pour générer des informations sensorielles intrinsèquement codées par impulsions selon l'une des revendications 1 et 2, où ladite impulsion est une impulsion électrique produite par un élément piézoélectrique (11, 30, 47, 69, 111) associé au dit élément mobile.

4. Procédé pour générer des informations sensorielles intrinsèquement codées par impulsions selon l'une quelconque des revendications précédentes, où ledit flux de chaleur positive est constitué par la chaleur naturelle du corps humain.

5. Procédé pour générer des informations sensorielles intrinsèquement codées par impulsions selon l'une quelconque des revendications 1 à 3, où ledit flux de chaleur positive est constitué par la chaleur produite par un équipement électrique et/ou électronique.

6. Procédé pour générer des informations sensorielles intrinsèquement codées par impulsions selon l'une quelconque des revendications précédentes, où le changement de section de l'ouverture de ladite chambre est produit par le glissement d'une coulisse (7, 63, 107) sur laquelle agit ledit stimulus mécanique externe (S), ledit glissement entraînant la déformation élastique de ladite chambre.

7. Procédé pour générer des informations sensorielles intrinsèquement codées par impulsions selon l'une quelconque de revendications 1 à 5, où le changement de section de l'ouverture de ladite chambre est provoqué par un glissement relatif élastiquement réversible de parois (21b, 41b) définissant ladite chambre, qui est produit par ledit stimulus mécanique externe.

8. Capteur biomimétique pour des applications robotiques et prothétiques, **caractérisé en ce qu'**il comporte une chambre élastiquement déformable (5, 32, 49, 66, 105) avec une ouverture (5a, 32a, 49a, 66a, 105a) dont la section varie en fonction de la déformation de ladite chambre et contenant un fluide humide qui peut se dilater sous l'effet d'un flux de chaleur positive (H) transmis par un corps (2) sur lequel est situé ledit capteur, un élément mobile (10, 29, 45, 67, 110) placé sur ladite ouverture et apte à se déplacer, alternativement et de façon répétitive, entre une première position, dans laquelle il est en contact avec le bord de ladite ouverture, et une seconde position, où il est éloigné de celui-ci, des moyens transducteurs (11, 30, 47, 69, 111) reliés au dit élément mobile et aptes à générer un train d'impulsions en réponse aux impacts correspondants du dit élément mobile contre le bord de ladite ouverture à la fin de son déplacement de retour vers ladite première position, une interface mobile (7, 21 b, 41 a, 63, 107) de ladite chambre sur laquelle agit un stimulus mécanique externe (S) dont l'intensité est proportionnelle à la variation de section de l'ouverture de ladite chambre, la dilatation du dit fluide produisant une augmentation de pression dans ladite chambre qui déplace ledit élément mobile de ladite première position à ladite seconde position, ledit élément mobile étant, pendant ce déplacement, supporté par un méat liquide capillaire (13, 33, 50, 72) qui croît graduellement entre lui et le bord de ladite ouverture jusqu'à atteindre un état d'instabilité capillaire dans ladite seconde position, dans laquelle ledit méat se casse, provoquant le brusque retour du dit élément mobile vers sa première position, fermant ainsi à nouveau ladite chambre et restaurant les conditions pour une nouvelle augmentation de pression dans celle-ci, la fréquence du train d'impulsions ainsi produit étant proportionnelle à la section de ladite ouverture et donc à l'intensité du stimulus mécanique externe qui agit sur ladite interface.

9. Capteur biomimétique selon la revendication 8, où ladite chambre (5, 105) est formée dans un corps (4, 104) d'un capteur présentant une symétrie sensiblement cylindrique, ladite ouverture (5a, 105a) étant réalisée à l'une de ses extrémités, et ladite interface étant constituée d'une coulisse se déplaçant le long de l'axe de ladite chambre reliée en glissement au dit corps de capteur de façon qu'un de ses mouvements vers ledit corps de capteur produise un changement de section de ladite ouverture.

10. Capteur biomimétique selon la revendication 9, où ledit corps de capteur (4, 104) comporte une couronne de bras divergents (6, 106) s'étendant à partir de son extrémité sur laquelle est réalisée ladite ouverture, ladite coulisse (7, 107) étant soutenue par lesdits bras en glissant entre eux.

11. Capteur biomimétique selon l'une quelconque de revendications 8 à 10, où lesdits moyens transducteurs comportent un élément piézoélectrique (11, 111) s'étendant à partir du dit élément mobile (10, 110).

12. Capteur biomimétique selon la revendication 11, où une cavité (8, 108) est formée sur ladite coulisse (7, 107), ledit élément piézoélectrique (11, 111) s'étendant dans cette cavité et constituant l'élément pour guider le glissement de ladite coulisse.

13. Capteur biomimétique selon l'une quelconque des revendications 9 à 12, où ladite coulisse (107) a une forme sensiblement hémisphérique, sur la surface externe de laquelle sont réalisées des nervures équidistantes (115) entre lesquelles sont disposés lesdits bras divergents (106).

14. Capteur biomimétique selon l'une quelconque des revendications 9 à 13, où lesdits bras divergents (6, 106) sont reliés élastiquement les uns aux autres.

15. Capteur biomimétique selon la revendication 8, où ladite chambre est formée dans un corps de capteur (21, 41) (22, 42) réalisé à l'aide d'une feuille de métal repliée en deux enroulements concentriques (21a, 21b, 41a, 41b) sur lesquels sont réalisées des fenêtres correspondantes respectives (26, 27, 43, 44) de façon à réaliser une ouverture (32a, 49a) de ladite chambre (32, 49) avec une section qui varie selon le glissement relatif des deux dits enroulements l'un par rapport à l'autre provoqué par le stimulus mécanique externe agissant sur ladite interface mobile, ledit élément mobile étant constitué d'une plaque articulée sur le bord de la fenêtre réalisée sur l'enroulement externe.

16. Capteur biomimétique selon la revendication 15, où ledit corps (21) d'un capteur (20) est fixé orthogonalement sur ledit corps (2) avec une extrémité axiale de l'enroulement externe (21b) et où ladite interface mobile (25b) est constituée du bord libre de l'extrémité axiale opposée de l'enroulement interne (21b), l'autre extrémité du dit enroulement interne étant éloignée du dit corps (2) pour permettre ledit glissement relatif, lesdites fenêtres (26, 27) étant orientées axialement sur lesdits enroulements.

17. Capteur biomimétique selon la revendication 15, où ledit corps (41) d'un capteur (40) est au contact du dit corps (2) le long de lune de ses génératrices, ladite interface mobile étant sensiblement formée par la génératrice diamétralement opposée, lesdites fenêtres (43, 44) étant orientées de façon circonférentielle sur lesdits enroulements (41 a, 41 b).

18. Capteur biomimétique selon la revendication 8, où ladite chambre (66) est formée dans un corps sensiblement semi-tubulaire (64) d'un capteur (60) qui peut être élastiquement déformé dans une direction axiale et relié rigidement, par ses extrémités, à une paire de supports parallèles et flexibles (61), ledit élément mobile étant une plaque (67) disposée sur une ouverture diamétrale (66a) du dit corps de capteur (64), ladite interface mobile étant une coulisse en coin (63) coopérant en glissement entre lesdits supports (61), de sorte qu'un mouvement de ladite coulisse vers ledit corps de capteur provoqué par ledit stimulus mécanique externe (S) entraîne l'éloignement des dits supports l'un par rapport à l'autre et un allongement axial du dit corps de capteur (64) avec une variation conséquente de la section de son ouverture diamétrale (66a).

19. Capteur biomimétique selon l'une quelconque des revendications 15 à 18, où lesdits moyens transducteurs comportent un élément piézoélectrique (30, 47, 69) relié au dit élément mobile (29, 45, 67).

20. Capteur biomimétique selon l'une quelconque des revendications précédentes, où une masse inertielle remplaçable (12, 31, 48, 70, 112) est associée au dit élément mobile.

21. Capteur biomimétique selon l'une quelconque des revendications précédentes, où des moyens de rappel élastiques (14, 28, 46, 68, 117) sont agencés entre ledit élément mobile et ledit corps de capteur.
